(19) Europäisches Patentamt
European Patent Office
Office européen des brevets

(11) Veröffentlichungsnummer: **0 088 943**
**B1**

(12) # EUROPÄISCHE PATENTSCHRIFT

(45) Veröffentlichungstag der Patentschrift: **13.08.86**

(51) Int. Cl.⁴: **A 61 K 9/08,** A 61 K 47/00, A 61 K 31/135

(21) Anmeldenummer: **83102015.1**

(22) Anmeldetag: **02.03.83**

(54) **Mittel zur Verbesserung der Resorption von injizierten antibakteriell wirksamen Substanzen und Kombinationen, enthaltend ein Benzylaminderivat.**

(30) Priorität: **16.03.82 DE 3209429**

(43) Veröffentlichungstag der Anmeldung: **21.09.83 Patentblatt 83/38**

(45) Bekanntmachung des Hinweises auf die Patenterteilung: **13.08.86 Patentblatt 86/33**

(84) Benannte Vertragsstaaten: **BE CH DE FR GB IT LI LU NL SE**

(56) Entgegenhaltungen:
**LU-A-34 976**
**NL-C-64 616**
**US-A-3 336 308**
**US-A-3 536 713**
**US-A-4 113 777**

**CHEMICAL ABSTRACTS, Band 82, Nr. 26, 30 Juni 1975, Seite 288, Nr. 175284r, Columbus, Ohio, USA M. CASTILLO COFINO et al.: "Spectrophotometric behavior of binary and ternary mixtures of solutions of chlophedimenol, bromhexine, cephalexin, and cephaloridine"**
**CHEMICAL ABSTRACTS, Band 96, Nr. 6, 8. Februar 1982, Seite 372, Nr. 40827p, Columbus, Ohio, USA W. BOWTIE et al.: "Bromhexine: in vitro and in vovo studies of release from mono- and bi-component preparations"**

(73) Patentinhaber: **BOEHRINGER INGELHEIM VETMEDICA GMBH, D-6507 Ingelheim/Rhein (DE)**

(72) Erfinder: **Kern, Otto, Dr., Rinderbachstrasse 33, D-6507 Ingelheim/Rhein (DE)**
Erfinder: **Wilhelm, Franz, Dr., Theodor- Fliedner-Strasse 12, D-6507 Ingelheim/Rhein (DE)**
Erfinder: **Salamon, Ernst, Dr., Kurpfalzstrasse 8, D-6507 Ingelheim/Rhein (DE)**

(74) Vertreter: **Laudien, Dieter, Dr., Boehringer Ingelheim Zentrale GmbH ZA Patente Postfach 200, D-6507 Ingelheim am Rhein (DE)**

EP 0 088 943 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Aus der Literatur ist bekannt, daß Benzylaminderivate in der Human- und Veterinärmedizin als Bronchosekretolytica eingesetzt werden. Die bekanntesten Vertreter dieser Benzylaminderivate sind hierbei das N-(2-Amino-3,5-dibrom-benzyl)-N-methyl-cyclohexylamin-hydrochlorid (Generic name: Bromhexine) und N-(2-Amino-3,5-dibrom-benzyl)-trans-4-hydroxy-cyclohexylamin-hydrochlorid (Generic name: Ambroxol). Diese Verbindungen erhöhen deutlich die Sekretmenge, außerdem wird eine Abnahme der Viskosität des Sekrets, eine Verminderung der Konzentration an Feststoffen in der Respirationstraktflüssigkeit und deren spezifischen Gewichts beobachtet, was die Benzylaminderivate als Sekretolytica charakterisiert.

Desweiteren ist literaturbekannt, daß bei oraler Gabe der obengenannten Benzylaminderivate zusammen mit einem Antibioticum, insbesondere mit Oxytetracyclin und Erythromycin, mit einem Sulfonamid, wie Sulfadiazin, die Einschleusung dieser Substanzen in das Bronchialsekret gesteigert wird. Gleiches trifft auch für körpereigene, nicht extra verabreichte Immunglobuline zu. Diese Steigerung der Konzentration der Bronchialsekretgehalte ist jedoch keine Folge einer durch die oben erwähnten Benzylaminderivate hervorgerufenen erhöhten Resorption aus dem Darm oder einer evtl. verzögerten Ausscheidung über die Niere, da sowohl nach oraler als auch intravenöser Gabe keine Erhöhung der Blutspiegelwerte beobachtet werden konnte.

Überraschenderweise wurde nun gefunden, daß bei parenteraler Verabreichung eines Benzylaminderivates der allgemeinen Formel

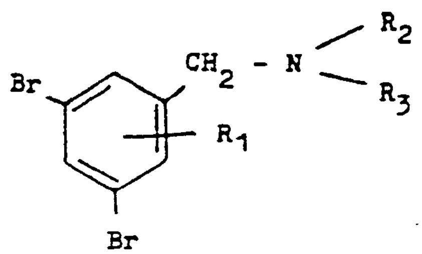

,(I)

und dessen physiologisch verträglicher Säureadditionssalze mit anorganischen oder organischen Säuren die Resorption einer parenteral ins Gewebe verabreichten allein nicht optimal resorbierbaren antibakteriell wirksamen Substanz oder Kombination beschleunigt wird. Erfindungsgemäß wird also durch die höheren Blutspiegel bei gleichbleibender Dosierung der antibakteriell wirksamen substanz oder Kombination ein besserer und sichererer therapeutischer Erfolg erzielt oder aber es kann — wird auf höhere Blutspiegel kein Wert gelegt — ihre Aufwandmenge im Vergleich zu ihrer bei alleiniger Verabreichung erforderlichen Menge gesenkt werden, das hießt, es wird eine nicht unwesentliche Einsparung erzielt. Außerdem wird die Rückstandproblematik entschärft, da bei den infrage kommenden antibakteriellen Substanzen und Kombinationen die Injektionsstelle üblicherweise das Gewebe ist, welches meßbare Rückstände dieser Substanzen am längsten enthält.

In der obigen allgemeinen Formel bedeutet

$R_1$ in 2- oder 4-Stellung eine Hydroxygruppe oder in 2-Stellung eine Aminogruppe,

$R_2$ ein Wasserstoffatom oder eine Alkylgruppe mit 1 bis 3 Kohlenstoffatomen und

$R_3$ eine gegebenenfalls durch eine Hydroxygruppe substituierte Cyclohexylgruppe.

Gegenstand der vorliegenden Erfindung ist somit die neue Verwendung der Benzylaminderivate der allgemeinen Formel I und deren physiologisch verträglichen Säureadditionssalze, vorzugsweise in der Veterinärmedizin, zur Herstellung eines parenteral ins Gewebe zu verabreichenden antibakteriellen Präparats mit verbesserter Resorption, enthaltend eine nicht optimal resorbierbare antibakteriell wirksame Substanz oder Kombination.

Bevorzugte Benzylaminderivate der obigen allgemeinen Formel I sind jedoch diejenigen, in denen $R_2$ und $R_3$ zusammen mit dem dazwischenliegenden Stickstoffatom die N-Methyl-cyclohexylamino-N-Äthyl-cyclohexylamino-, trans-4-Hydroxy-cyclohexylamino- oder cis-3-Hydroxy-cyclohexylaminogruppe darstellen.

Besonders bevorzugte Benzlaminderivate der obigen allgemeinen Formel I stellen jedoch die Verbindung N-(3,5-Dibrom-2-hydroxy-benzyl)-trans-4-hydroxy-cyclohexylamin und deren physiologisch verträgliche Säureadditionssalze mit anorganischen oder organischen Säuren, insbesondere deren Hydrochlorid dar.

Als antibakterielle substanzen, wobei auch gegebenenfalls deren Ester oder Salze eingesetzt werden können, kommen erfindungsgemäß beispielsweise folgende in Betracht:

ein Antibioticum der Tetracyclingruppe, z.B. Oxytetracyclin, Oxytetracyclin-hydrochlorid, Rolitetracyclin oder Doxycyclin, ein schwerer lösliches Penicillin, z.B. Procain-Penicillin, Benethamin-Penicillin, Benzathin-Penicillin, die Benzathin-Salze von Oxacillin, Cloxacillin oder Ampicillin, Erythromycin und seine Derivate wie beispielsweise 9-Deoxy-11-deoxy-9,11-[imino-[2-(2-methoxyäthoxy)-äthyliden]oxy]-(9F)-erythromycin, Erythromycin-lactobionat, Erythromycin-äthylsuccinat, Erythromycin-glucoheptonat, Spiramycin, Spiramycin-adipat, Tylosin, Tylosin-tartrat, Oleandomycin, Chloramphenicol, Chloramphenicol-succinat, Thiamphenicol, Thiamphenicol-glycinat oder

ein Sulfonamid oder dessen Natriumsalz, z.B. Sulfadiazin, Sulfadoxin, Sulfamethoxazol, Sulfadimethoxin, Sulfadimidin oder Sulfathiazol, oder eine Sulfonamid-Kombination mit einem Agonisten wie Trimethoprim, z.B. die Sulfadimidin-Sulfathiazol-Trimethoprim-Kombination oder die entsprechende Natriumsalz-Kombination,

sowie gegebenenfalls deren entsprechende Depotformen.

Ein weiterer Gegenstand der vorliegenden Erfindung sind die neuen ins Gewebe parenteral applizierbaren Kombinationen, enthaltend ein Benzylaminderivat der obigen allgemeinen Formel I und eine der oben genannten allein nicht optimal resorbierbaren antibakteriell wirksamen Substanzen oder Kombinationen neben einem oder mehreren üblichen inerten Verdünnungsmitteln oder Trägerstoffen, vorzugsweise deren für die intramuskuläre Verabreichung geeignete Applikationsformen.

Bevorzugte Kombinationen sind hierbei diejenigen, welche ein Benzylaminderivat der obigen allgemeinen Formel I, in der $R_1$ eine Hydroxygruppe in 2- oder 4-Stellung und $R_2$ und $R_3$ zusammen mit dem dazwischenliegenden Stickstoffatom die vorstehend erwähnten Bedeutungen besitzen, zweckmäßigerweise jedoch die N-Äthylcyclohexylamino-, trans-4-Hydroxycyclohexylamino- oder cis-3-Hydroxy-cyclohexylaminogruppe, vorzugsweise jedoch $R_1$ in 2-Stellung die Hydroxygruppe und $R_2$ und $R_3$ zusammen mit dem dazwischenliegenden Stickstoffatom die trans-4-Hydroxy-cyclohexylaminogruppe darstellen, und eine der vorstehend erwähnten antibakteriell wirksamen Substanzen oder Kombinationen enthalten, oder eine Kombination bestehend aus

N-(2-Amino-3,5-dibrom-benzyl)-N-methyl-cyclohexyamin sowie dessen physiologisch verträglichen Säureadditionssalzen und einem Oxytetracyclin-Langzeitpräparat, Oxytetracyclin-hydrochlorid-Langzeitpräparat, Rolitetracyclin, Doxycyclin,

einem schwerer löslichen Penicillin, z.B. Procain-Penicillin, Benethamin-Penicillin, Benzathin-Penicillin, einem Benzathin-Salz von Oxacillin, Cloxacillin oder Ampicillin,

Erythromycin oder einem seiner Derivate wie beispielsweise 9-Deoxy-11-deoxy-9,11-[imino-[2-(2-methoxyäthoxy)-äthyliden]oxy]-(9F)-erhthromycin, Erythromycin-lactobionat, Erythromycin-äthylsuccinat, Erythromycin-glucoheptonat, Spiramycin, Spiramycin-adipat, Tylosin, Tylosin-tartrat, Oleandomycin, Thiamphenicol, Thiamphenicol-glycinat oder

einem Sulfonamid oder dessen Natriumsalz wie Sulfadiazin, Sulfadoxin, Sulfamethoxazol, Sulfadimethoxin, Sulfadimidin oder Sulfathiazol oder einer Sulfonamid-Kombination mit einem Agonisten wie Trimethoprim, z.B. die Sulfadimidin-Sulfathiazol-Trimethoprim-Kombination oder eine Kombination bestehend aus

N-(2-amino-3,5-dibrom-benzyl)-trans-4-hydroxy-cyclohexylamin sowie dessen physiologisch verträglichen Säueradditionssalzen und einem Antibioticum der Tetracyclingruppe, z.B. Oxytetracyclin, Oxytetracyclin-hydrochlorid, Rolitetracyclin oder Doxycyclin,

einem schwer löslichen Penicillin, z.B. Procain-Penicillin, Benethamin-Penicillin, Benzathin-Penicillin, einem Benzathin-Salz von Oxacillin, Cloxacillin oder Ampicillin, Erythromycin oder einem seiner Derivate wie beispielsweise 9-Deoxy-11-deoxy-9,11-[imino[2-(2-methoxyäthoxy)-äthyliden]oxy]-(9F)-erythromycin, Erythromycin-lactobionat, Erythromycinäthylsuccinat, Erythromycin-glucoheptonat, Spiramycin, Spiramycin-adipat, Tylosin, Tylosin-tartrat, Oleandomycin, Chloramphenicol, Chloramphenicol-succinat, Thiamphenicol, Thiamphenicol-glycinat oder einem

Sulfonamid oder dessen Natriumsalz, z.B. Sulfadiazin, Sulfadoxin, Sulfamethoxyazol, Sulfadimethoxin, Sulfadimidin oder Sulfathiazol, oder einer Sulfonamid-Kombination mit einem Agonisten wie Trimethoprim, z.B. die Sulfadimidin-Sulfathiazol-Trimethoprim-Kombination, sowie gegebenenfalls deren entsprechende Depotformen.

Besonders bevorzugte Kombinationen stellen jedoch folgende dar:

Kombinationen von N-(2-Amino-3,5-dibrom-benzyl)-N-methyl-cyclohexylamin sowie dessen physiologisch verträglichen Säureadditionssalzen oder N-(3,5-Dibrom-2-hydroxy-benzyl)-trans-4-hydroxy-cyclohexylamin sowie dessen physiologisch verträglichen Säureadditionssalzen mit Erythromycin, Erythromycin-lactobionat, Erythromycin-äthylsuccinat, Erythromycin-glucoheptonat, 9-Deoxy-11-deoxy-9,11-[imino[2-(2-methoxyäthoxy)-äthyliden]oxy]-(9F)-erythromycin, Tylosin, Tylosin-tartrat, Spiramycin, Spiramycin-adipat, Oleandomycin, Benzathin-Penicillin, Benethamin-Penicillin, Ampicillin, Oxacillin, Cloxacillin, Rolitetracyclin, Doxycyclin und ihren Salzen, sowie einem Sulfonamid und seinen Salzen, auch in Kombination mit Trimethoprim,

sowie Kombinationen von N-(3,5-Dibrom-2-hydroxy-benzyl)-trans-4-hydroxy-cyclohexylamin und dessen physiologisch verträglichen Säureadditionssalzen mit Oxytetracyclin und seinen Salzen, Chloramphenicol, Chloramphenicol-succinat, Thiamphenicol oder Thiamphenicol-glycinat.

Beispielsweise wurde die resorptionsfördernde Wirkung der Benzylaminderivate

A = N-(2-Amino-3,5-dibrom-benzyl)-N-methyl-cyclohexylamin-hydrochlorid,

B = N-(2-Amino-3,5-dibrom-benzyl)-trans-4-hydroxy-cyclohexylamin-hydrochlorid und

C = N-(3,5-Dibrom-2-hydroxy-benzyl)-trans-4-hydroxy-cyclohexylamin-hydrochlorid

wie folgt geprüft:

Rinder, Schweine und Schafe — jeweils die gleichen 10 Tiere pro Gruppe — wurden einmal nur mit der betreffenden antibakteriellen Substanz bzw. Kombination, einmal mit der Kombination des

Benzylaminderivates mit der gleichen antibakteriellen Substanz bzw. Kombination intramuskulär behandelt. Beide Behandlungen erfolgten im Abstand von 8 Tagen, um die totale Ausscheidung des bzw. der bei der ersten Gabe verabreichten Stoffe zu gewährleisten. Die Reihenfolge der Behandlungen war dabei variabel, d.h. z.T. wurde als erstes die antibakterielle Substanz bzw. Kombination (Kontrolle), z.T. die Kombination mit dem Benzylaminderivat verabreicht (Versuchsgruppe). In einigen Fällen wurden die Versuche in "Cross over-Anordnung" durchgeführt, d.h. beim ersten Behandlungstermin erhielten 5 Tiere die antibakterielle Substanz bzw. Kombination, 5 Tiere die Kombination mit dem Benzylaminderivat. Bei der Wiederholung 8 Tage später war die Behandlung der Tiere umgekehrt.

Blutproben wurden tagsüber in 1- bzw. 2-stündigen Abständen und nach 24 Stunden genommen, in einigen Fällen auch noch nach 32 Stunden, in 2 Fällen (Langzeitpräparat) auch nach 48 und 72 Stunden. Die Ermittlung der Antibiotika- bzw. Sulfonamid-Gehalte des Blutserums erfolgte nach allgemein üblichen mikrobiologischen Verfahren mit für jede Substanz spezifischen Testkeimen. Verglichen wurden als Gesamtmaß der antibakteriellen Aktivität jeweils die Flächen unter den erhaltenen Blutspiegelkurven. Dies ergab für die Kombination der Benzylaminderivate mit der betreffenden antibakteriellen Substanz bzw. Kombination folgende Blutspiegel-Steigerungen gegenüber der jeweiligen Kontrollgruppe:

| Antibakteriell wirks. Subst./ Kombination<br>Dosis mg/kg Kgw | | Benzylamin-Derivat<br>Dosis mg/kg | Tierart | Blutspiegel-Steigerung in % gegenüber Kontrolle |
|---|---|---|---|---|
| Erythromycin | 10 | 0,3 i.m. A | Rind | 16,1 |
| | 10 | 0,6 i.m. C | Rind | 21,4 |
| | 10 | 0,6 i.m. A | Schwein | 59,1 |
| | 10 | 1,2 i.m. A | Schwein | 31,4 |
| Erythromycin-Derivat | 10 | 1,2 i.m. A | Schwein | 15,3 |
| | 10 | 1,2 i.m. C | Schwein | 45,4 |
| Oxytetracyclin-hydrochlorid | 10 | 0,6 i.m. C | Rind | 16,3 |
| Oxytetracyclin[1) ] Depotpräparat | 20 | 0,6 i.m. A | Schwein | 109,5 |
| | 20 | 1,2 i.m. C | Schwein | 80,0 |
| Tylosin | 15 | 0,3 i.m. A | Rind | 33,0 |
| | 15 | 0,6 i.m. C | Rind | 23,5 |
| | 10 | 0,6 i.m. A | Schwein | 19,3 |
| | 10 | 1,2 i.m. A | Schwein | 30,3 |
| | 10 | 0,6 i.m. C | Schwein | 30,5 |
| | 10 | 1,2 i.m. C | Schwein | 26,1 |
| Sulfadimidin- | 24 | 0,6 i.m. A | Schwein | 29,6 |
| Sulfathiazol- | 24 | 1,2 i.m. A | Schwein | 25,1 |
| Trimethoprim- | 24 | 0,6 i.m. C | Schwein | 29,9 |
| Kombination (10:10:4) | 24 | 0,6 i.m. C | Schwein | 20,3 |

[1)] Blutspiegel über 72 h verfolgt; nach 48 h 0,13 µg/ml für Kontrolle, aber 0,35 µg/ ml für Versuchsgruppe; nach 72 h Kontrolle 0,00 µg/ml, Versuchsgruppe noch 0,19 µg/ml.

Die erfindungsgemäß verwendeten Benzylaminderivate der allgemeinen Formel I sowie ihre physiologisch verträglichen Säureadditionssalze mit anorganischen oder organischen Säuren weisen eine gute Verträglichkeit auf. So beträgt beispielsweise an der Maus die akute Toxizität ($LD_{50}$) der

| | |
|---|---|
| Substanz A | >400 mg/kg i.p., |
| Substanz B | 268 mg/kg i.p. und |
| Substanz C | >800 mg/kg i.p. |

Aufgrund der vorstehend erwähnten biologischen Daten eignen sich die Benzylaminderivate der allgemeinen Formel I und deren physiologisch verträgliche Säureadditionssalze, wie bereits eingangs erwähnt, zur Verbesserung der Resorption von parenteral ins Gewebe applizierten antibakteriell wirksamen Substanzen oder Kombinationen und damit zur Verbesserung und Sicherung des therapeutischen Erfolges. Die Dosierung liegt hierbei zweckmäßigerweise oberhalb 0,1 mg/kg, vorzugsweise jedoch zwischen 0,2 und 2,0 mg/kg, und wird in Lösungen nach oben durch die Löslichkeit des verwendeten Benzylaminderivates begrenzt. So beträgt beispielsweise in Wasser je nach pH im sauren Bereich die maximale Löslichkeit der

| | |
|---|---|
| Substanz A | 0,2—5,0 $mg/cm^3$ |
| Substanz B | 16,6 $mg/cm^3$ und |
| Substanz C | 0,1—1,0 $mg/cm^3$. |

In öligen Vehikeln können je nach der Öllöslichkeit des Benzylaminderivates selbstverständlich auch höhere Konzentrationen erzielt werden, ebenso, wenn das Benzylaminderivat in geeigneten Vehikeln, in denen es nicht oder nicht ausreichend löslich ist, suspendiert wird.

Ferner erfolgt die Applikation des Benzylaminderivates zweckmäßigerweise gleichzeitig mit einer therapeutischen Dosis der zu applizierenden antibakteriell wirksamen Substanz oder Kombination.

So beträgt beispielsweise die Einzeldosis an Oxytetracyclin 5—30 mg/kg, Oxytetracyclin-hydrochlorid 2—25 mg/kg, Rolitetracyclin 15—50 mg/kg, Doxycyclin 2—5 mg/kg, Erythromycin 5—20 mg/kg, 9-Deoxy-11-deoxy-9,11-[imino[2-(2-methoxyäthoxy)äthyliden]oxy]-(9F)-erythromycin 5—20 mg/kg, Spiramycin 10—50 mg/kg, Tylosin 5—20 mg/kg, Chloramphenicol 10—50 mg/kg, Thiamphenicol 10—50 mg/kg, Sulfadiazin 15—50 mg/kg, Sulfadiazin-Sulfathiazol-Trimethoprim 15—30 mg/kg, Sulfadoxin-Trimethoprim 15—30 mg/kg, Procain-Penicillin 2 000—20 000 I.E./kg, Benzathin-Pencillin 6 000—25 000 I.E./kg, Ampicillin 2—15 mg/kg, Oxacillin 5—15 mg/kg oder Cloxacilin 5—15 mg/kg.

Geeignete Darreichungsformen stellen beispielsweise folgende dar:

Injektionszubereitungen, wäßriger, wassermischbarer oder öliger Natur, in denen die betreffenden antibakteriellen Substanzen in praxisgerechter Konzentration gelöst oder suspendiert sind. Das gleiche gilt, je nach ihrer Löslichkeit, für die Benzylaminderivate oder ihre Salze, wobei in der gleichen Zubereitung auch die eine substanz gelöst und die andere suspendiert vorliegen kann. In Fällen, in denen eine wäßrige Lösung erwünscht, aus Gründen ungenügender Stabilität, z.B. des Antibiotikums, aber nicht zweckmäßig ist, wird die injektionsfertige Kombination erst kurz vor der Applikation durch Lösen oder Suspendieren von Trockensubstanz in dem das Benzylaminderivat enthaltenen Lösungsmittel hergestellt.

Die erfindungsgemäß eingesetzten Benzylaminderivate der allgemeinen Formel I und deren physiologisch verträgliche Säureadditionssalze sind literaturbekannt (siehe US-Patenschriften 3 336 308, 3 536 713 und 4 113 777).

Die nachfolgenden Beispiele sollen die Erfindung näher erläutern:

Beispiel 1

Zweikompartiment-Präparat mit 9-Deoxy-11-deoxy-9,11-[imino-[2-(2-methoxyäthoxy)-äthyliden]oxy]-(9F)-erythromycin-lactobionat und N-(2-Amino-3,5-dibrom-benzyl)-N-methyl-cyclohexylamin-hydrochlorid (wäßrige Lösung)

Zusammensetzung

a) Trockenampulle

| | |
|---|---|
| Antibioticum | 715,0 mg |

b) Lösungsmittelampulle

| | |
|---|---|
| Wirksubstanz | 75,0 mg |
| Weinsäure | 37,5 mg |
| Glycerin-Polyethylen-glykoloxystearat | 250,0 mg |
| Glucose | 200,0 mg |
| Aqua pro injectione ad | 5,0 ml |

Herstellung

Zur Herstellung der Trockenampulle wird der Wirkstoff in Wasser für Injektionszwecke gelöst, über ein geeignetes Filtersystem, gegebenenfalls unter Einsatz von Pyrogenadsorptionsschichten, steril und pyrogenfrei gemacht und unter aseptischen Bedingungen in den gewünschten Dosierungen in gereinigte und sterilisierte 10 ml-Injektionsfläschchen abgefüllt. Die Gefriertrocknung dieser Fläschchen erfolgt in üblicher Weise.

Zur Herstellung der Lösungsampullen werden der Wirkstoff und die Hilfsstoffe nacheinander in Wasser für Injektionszwecke gelöst, in analoger Weise wie die Trockenampullenlösung filtriert und in 5 ml-Ampulen abgefüllt. Zur Sterilisation werden die zugeschmolzenen Ampullen bzw. die mit Gummistopfen und Aluminiumbördelkappen verschlossenen Injektionsfläschchen 20 Minuten auf 121°C erhitzt.

Beispiel 2

Zweikompartiment-Präparat mit 9-Deoxy-11-deoxy-9,11-[imino-[2-(2-methoxyäthoxy)-äthyliden]oxy]-(9F)-erythromycin-lactobionat und N-(2-Amino-3,5-dibrom-benzyl)-N-methyl-cyclohexylamin-hydrochlorid (wäßrige Lösung)

Zusammensetzung

a) Trockenampulle

| | |
|---|---|
| Antibioticum | 3 575,0 mg |

b) Lösungsmittelampulle

| | |
|---|---|
| Wirksubstanz | 375,0 mg |
| Weinsäure | 187,5 mg |
| Glycerin-Polyethylen-glykoloxystearat | 1 250,0 mg |
| Glucose | 1 000,0 mg |
| Aqua pro injectione ad | 25,0 ml |

Herstellung

analog Beispiel 1

Die Abfüllung erfolgt jedoch in 25 ml- bzw. 30 ml-Injektions-fläschchen.

Beispiel 3

Ölige Suspension, enthaltende 9-Deoxy-11-deoxy-9,11-[imino-[2-(2-methoxyäthoxy)-äthyliden]oxy]-(9F)-erythromycin und N-(3,5-Dibrom-2-hydroxy-benzyl)-trans-4-cyclohexylamin-hydrochlorid

Zusammensetzung

| | |
|---|---|
| Antibioticum | 500,0 mg |
| Wirksubstanz | 100,0 mg |
| Benzylalkohol | 50,0 mg |
| Neutralöl ad | 5,0 ml |

Herstellung

Das Antibioticum und die Wirksubstanz werden im Gemisch der beiden Hilfstoffe unter Erwärmen gelöst bzw. suspendiert und unter aseptischen Bedingungen in gereinigte und sterilisierte 5 ml-Ampullen abgefüllt.

Beispiel 4

Ölige Suspension, enthaltende 9-Deoxy-11-deoxy-9,11-[imino-[2-(2-methoxyäthoxy)-äthyliden]oxy]-(9F)-erythromycin und N-(3,5-Dibrom-2-hydroxy-benzyl)-trans-4-cyclohexylamin-hydrochlorid

Zusammensetzung

| | |
|---|---|
| Antibioticum | 2 500,0 mg |
| Wirksubstanz | 500,0 mg |
| Benzylalkohol | 250,0 mg |
| Neutralöl ad | 25,0 ml |

Herstellung

Das Antibioticum und die Wirksubstanz werden im Gemisch der beiden Hilfstoffe unter Erwärmen gelöst bzw. suspendiert und unter aseptischen Bedingungen in gereinigte und sterilisierte 25 ml-Injektionsfläschchen abgefüllt.

Beispiel 5

Zweikompartiment-Präparat mit 9-Deoxy-11-deoxy-9,11-[imino-[2-(2-methoxyäthoxy)-äthyliden]oxy]-(9F)-erythromycin-lactobionat und N-(3,5-Dibrom-2-hydroxy-benzyl)-trans-4-hydroxy-cyclohexylamin-hydrochlorid (wäßrige Suspension)

Zusammensetzung

a) Trockenampulle

| | |
|---|---|
| Antibioticum | 715,0 mg |

b) Suspensionslösungsampulle

| | |
|---|---|
| Wirksubstanz | 100,0 mg |
| Polyethylenglycolstearat | 1,0 mg |
| Sorbit | 250,0 mg |
| Methylhydroxyäthylcellulose | 15,0 mg |
| Aqua pro injectione ad | 5,0 ml |

Herstellung

Zur Herstellung der Trockenampulle wird der Wirkstoff in Wasser für Injektionszwecke gelöst, über ein geeignetes Filtersystem, gegebenenfalls unter Einsatz von Pyrogenadsorptionsschichten, steril und pyrogenfrei gemacht und unter aspetischen Bedingungen in den gewünschten Dosierungen in gereinigte

und sterilisierte 10 ml-Injektionsfläschchen abgefüllt. Die Gefriertrocknung dieser Fläschchen erfolgt in üblicher Weise.

Zur Herstellung der Suspensions-Lösungsampullen werden die Hilfsstoffe in Wasser für Injektionszwecke gelöst und die Lösung steril und gegebenenfalls pyrogenfrei filtriert. In dieser Lösung wird unter aseptischen Bedingungen der Wirkstoff einsuspendiert und die Suspension unter Rühren in gereinigte und sterilisierte 5 ml-Ampullen abgefüllt.

Beispiel 6

Zweikompartiment-Präparat mit 9-Deoxy-11-deoxy-9,11-[imino[2-(2-methoxyäthoxy)-äthyliden]oxy]-(9F)-erythromycin-lactobionat und N-(3,5-Dibrom-2-hydroxy-benzyl)-trans-4-hydroxy-cyclohexylamin-hydrochlorid (wäßrige Suspension)

Zusammensetzung

a) Trockenampulle

| | |
|---|---|
| Antibioticum | 3 575,0 mg |

b) Suspensionlösungsampulle

| | |
|---|---|
| Wirksubstanz | 500,0 mg |
| Polyethylenglycolstearat | 5,0 mg |
| Sorbit | 1 250,0 mg |
| Methylhydroxyäthylcellulose | 75,0 mg |
| Aqua pro injectione ad | 25,0 ml |

Herstellung

analog Beispiel 5

Die Abfüllung erfolgt jedoch in 30 ml- bzw. 25 ml-Injektionsfläschchen.

Beispiel 7

Injektionslösung, enthaltend Oxytetracyclin-hydrochlorid und N-(3,5-Dibrom-2-hydroxy-benzyl)-trans-4-hydroxy-cyclohexylamin·

Zusammensetzung

| | |
|---|---|
| Antibioticum x HCl | 5,0 g |
| Wirksubstanz | 0,05—0,8 g |
| Magnesiumoxid | 0,45 g |
| pH-Korrigenz | 1,0 g |
| Antioxidantien | 0,2 g |
| Solketal | 15,0 g |
| 1,2-Propylenglycol | 74,0 g |
| Aqua pro injectione ad | 100,0 ml |

Herstellung

In einem geeigneten Gefäß wird das Antibioticum in der entsprechenden Menge Wasser und 1,2-Propylenglycol gelöst und Magnesiumoxid zugesetzt. Parallel dazu stellt man eine Lösung von Solketal und Wirksubstanz in der entsprechenden 1,2-Propylenglycolmenge her. Die beiden Wirkstofflösungen werden vereinigt und zugesetzt eine Lösung der Antioxidantien in wenig Wasser. Mit pH-Korrigenz wird der gewünschte pH-Wert eingestellt. Fertigung und Abfüllung sind sowohl unter $N_2$-Begasung als auch aseptischen Bedingungen durchzuführen. Sterilfiltration ist notwendig.

Beispiel 8

Wässrige Suspension, enthaltend Chloramphenicol oder Thiamphenicol und N-(3,5-Dibrom-2-hydroxybenzyl)-trans-4-hydroxy-cyclohexylamin-hydrochlorid

Zusammensetzung

| | |
|---|---|
| Antibioticum | 20,0 g |
| Wirksubstanz | 0,05—2,5 g |
| Suspensionsstabilisatoren | 1,6 g |
| Emulgator | 2,0 g |
| Zitronensäure | 1,0 g |
| Antischaummittel | 0,2 g |
| Thimerosal | 0,005 g |
| 1 n Natronlauge | 3,25 g |
| Aqua pro injectione ad | 100,00 ml |

Herstellung

Thimerosal und Zitronensäure werden in ca. einem Drittel der Wassermenge gelöst und in einem geeigneten Gefäß vorgelegt. In diese Lösung wird in der Reihenfolge Wirksubstanz, Suspensionsstabilisatoren und Antischaummittel zugegeben und gelöst bzw. suspendiert.

Der Emulgator wird unter Erwämen in ca. 1/3 der Wassermenge gelöst und zugegeben. Unter Rühren wird eine Suspension von Chloramphenicol in Wasser zugefügt, bei der homogenen Suspension wird mit 1 n NaOH der geeignete pH-Wert eingestellt, mit dem restlichen Wasser wird vorher auf 100 ml aufgefüllt. Sämtliche Hilfs- und Wirkstoffe bzw. deren Lösungen werden vor deren Verwendung sterilisiert. Die Fertigung bzw. Abfüllung muß unter aseptischen Bedingungen erfolgen.

Beispiel 9

Injectionslösung, ethaltend Tylosin und N-(2-Amino-3,5-dibrombenzyl)-N-methyl-cyclohexylamin-hydrochlorid

Zusammensetzung

| | |
|---|---|
| Tylosin | 50,0 mg |
| Wirksubstanz | 0,5—6,0 mg |
| 1,2-Propylenglycol | 0,5 ml |
| Benzylalkohol | 0,04 ml |
| Salzsäure ad pH 4 | |
| Aqua pro injectione ad | 1,0 ml |

Herstellung

In 90 ml eines entsprechenden 1,2-Propylenglycol-Wassergemisches wird unter Rühren sowie Ultraschallbehandlung Wirksubstanz unter $N_2$-Begasung gelöst. Tylosin wird zugesetzt und klar gelöst. Nach Zufügen des Benzylalkohols wird mit 1 n HCl der gewünschte pH-Wert eingestellt. Mit Wasser wird auf 100 ml aufgefüllt.

Fertigung und Abfüllung müssen unter aseptischen Bedingungen vorgenommen werden.

Beispiel 10

Injektionslösung, enthaltend Tylosin und N-(3,5-Dibrom -2-hydroxybenzyl)-trans-4-hydroxy-cyclohexylamin-hydrochlorid

Zusammensetzung

| | |
|---|---|
| Tylosin | 50,0 mg |
| Wirksubstanz | 0,5—6,0 mg |
| 1,2-Propylenglycol | 0,5 ml |
| Benzylalkohol | 0,04 ml |
| Salzsäure ad pH 4 | |
| Aqua pro injectione ad | 1,0 ml |

Herstellung
analog Beispiel 9.

Beispiel 11

Ölsuspension, enthaltend Erythromycin und N-(3,5-Dibrom-2-hydroxy-benzyl)-trans-4-hydroxy-cyclohexylamin-hydrochlorid

Zusammensetzung

| | |
|---|---|
| Erythromycin | 50,0 mg |
| Wirksubstanz | 0,5—25,0 mg |
| Dioctylsulfosuccinat-Natrium | 2,0 mg |
| Neutralöl ad | 1,0 ml |

Herstellung

In der entsprechenden Menge Neutralöl wird Dioctylsulfosuccinat-Natrium unter Erwärmen und Rühren gelöst. Nach Abkühlen auf Raumtemperatur wird Erythromycin gelöst und Wirksubstanz geeigneter Teilchengröße zugegeben. Die resultierende Suspension wird mit einem geeigneten Rührwerk homogenisiert und unter aseptischen Bedingungen abgefüllt.

Beispiel 12

Suspension, enthaltend Erythromycin und N-(2-Amino-3,5-dibrombenzyl)-N-methyl-cyclohexylamin

Zusammensetzung

| | |
|---|---|
| Erythromycin | 50,0 mg |
| Wirksubstanz | 0,5—25,0 mg |
| Neutralöl | 1,0 ml |

Herstellung
analog Beispiel 11.

## Beispiel 13

Injektionslösung, enthaltend Trimethoprim, Sulfadimidin, Sulfathiazol und N-(2-Amino-3,5-dibrom-benzyl)-N-methyl-cyclohexylamin-hydrochlorid

Zusammensetzung

| | |
|---|---|
| Trimethoprim | 40,0 mg |
| Sulfadimidin | 100,0 mg |
| Sulfathiazol | 100,0 mg |
| Wirksubstanz | 0,5—6,0 mg |
| Glycerinformal ad | 1,0 ml |

Herstellung

Wirksubstanz wird unter $N_2$-Begasung in Glycerinformal unter Rühren gelöst. Nacheinander werden unter Rühren Trimethoprim, Sulfadimidin und Sulfathiazol gelöst. Anschließend wird mit restlichem Glycerinformal aufgefüllt.

Fertigung und Abfüllung sind unter aseptischen Bedingungen und Lichtschutz durchzuführen.

## Beispiel 14

Injektionslösung, enthaltend Trimethoprim, Sulfadimidin, Sulfathiazol und N-(3,5-Dibrom-2-hydroxy-benzyl)-trans-4-hydroxycyclohexylamin-hydrochlorid

Zusammensetzung

| | |
|---|---|
| Trimethoprim | 40,0 mg |
| Sulfadimidin | 100,0 mg |
| Sulfathiazol | 100,0 mg |
| Wirksubstanz | 0,5—15,0 mg |
| Glycerinformal ad | 1,0 ml |

Herstellung

analog Beispiel 13.

## Beispiel 15

Injektionslösung, enthaltend Spiramycin und N-(2-Amino-3,5-dibrom-benzyl)-trans-4-hydroxy-cyclohexylamin-hydrochlorid

Zusammensetzung

| | |
|---|---|
| Wirksubstanz | 0,5—6,0 mg |
| Spiramycin | 50,0 mg |
| 1,2-Propylenglycol | 0,5 ml |
| Aqua pro injectione ad | 1,0 ml |
| 1 n Salzsäure ad | 3,8 |

Herstellung

Wirksubstanz wird unter $N_2$-Begasung in 1,2-Propylenglycol/Wasser-Gemisch unter Rühren gelöst. Spiramycin wird ebenso gelöst. Mit 1 n HCl wird der gewünschte pH-Wert eingestellt und mit Wasser aufgefüllt.

Fertigung und Abfüllung sind unter aseptischen Bedingungen durchzuführen.

Beispiel 16

Injektionslösung, enthaltend Spiramycin und N-(3,5-Dibrom-2-hydroxy-benzyl)-trans-4-hydroxy-cyclohexylamin

Zusammensetzung

| | |
|---|---|
| Wirksubstanz | 0,5—15,0 mg |
| Spiramycin | 50,0 mg |
| Glucofural ad | 1,0 ml |

Herstellung

In der unter $N_2$-Begasung hergestellten Lösung aus Spiramycin in Glucofural wird Wirksubstanz portionsweise unter weiterer $N_2$-Begasung eingetragen und gelöst.

Die Abfüllung erfolgt unter aseptischen Bedingungen sowie $N_2$-Begasung.

Beispiel 17

Zweikompartiment-Präparat mit 9-Deoxy-11-deoxy-9,11-[imino[2-(2-methoxyäthoxy)-äthyliden]oxy]-(9F)-erythromycin-lactobionat und N-(2-Amino-3,5-dibrom-benzyl)-trans-4-hydroxy-cyclohexylamin-hydrochlorid

Zusammensetzung

a) Trockenampulle

| | |
|---|---|
| Antibioticum | 715,0 mg |

b) Lösungsmittelampulle

| | |
|---|---|
| Wirksubstanz | 75,0 mg |
| Weinsäure | 37,5 mg |
| Glycerin-Polyethylenglycoloxystearat | 250,0 mg |
| Glucose | 200,0 mg |
| Aqua pro injectione ad | 5,0 ml |

Herstellung

analog Beispiel 1.

Selbstverständlich lassen sich alle in der Veterinärmedizin gebräuchlichen schwerer resorbierbaren antibakteriellen Substanzen oder Kombinationen, welche vorstehend nicht ausdrücklich genannt wurden, in die enstprechenden üblichen Darreichungsformen zusammen mit einem Benzylaminderivat einarbeiten.

**Patentansprüche**

1. Verwendung eines Benzylaminderivates der allgemeinen Formel

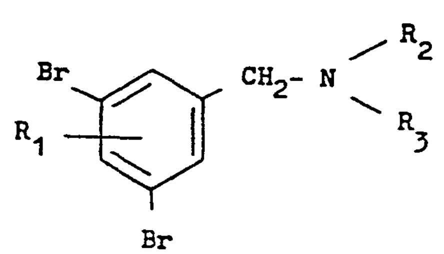

, (I)

in der

$R_1$ in 2- oder 4-Stellung eine Hydroxygruppe oder in 2-Stellung eine Aminogruppe,

$R_2$ ein Wasserstoffatom oder eine Alkylgruppe mit 1 bis 3 kohlenstoffatomen und

$R_3$ eine gegebenenfalls durch eine Hydroxygruppe substituierte Cyclohexylgruppe darstellen, oder ein physiologisch verträgliches Säureadditionssalz hiervon mit einer anorganischen oder organischen Säure zur Herstellung eines parenteral ins Gewebe zu verabreichenden antibakteriellen Präparats mit verbesserter Resorption, enthaltend eine nicht optimal resorbierbare antibakteriell wirksame Substanz oder Kombination.

2. Verwendung eines Benzylaminderivates der allgemeinen Formel I gemäß Anspruch 1, dadurch gekennzeichnet, daß $R_1$ wie im Anspruch 1 definiert ist,

$R_2$ und $R_3$ zusammen mit dem dazwischenliegenden Stickstoffatom die N-Methyl-cyclohexylamino-, N-Äthyl-cyclohexylamino-, trans-4-Hydroxy-cyclohexylamino- oder cis-3-Hydroxy-cyclohexylaminogruppe darstellen.

3. Verwendung eines Benzylaminderivates der allgemeinen Formel I gemäß Anspruch 1, dadurch gekennzeichnet, daß

$R_1$ in 2-Stellung die Hydroxygruppe,

$R_2$ und $R_3$ zusammen mit dem dazwischenliegenden Stickstoffatom die trans-4-Hydroxy-cyclohexylaminogruppe darstellen.

4. Kombination zur parenteralen Verabreichung ins Gewebe, enthaltend

ein Benzylaminderivat der allgemeinen Formel

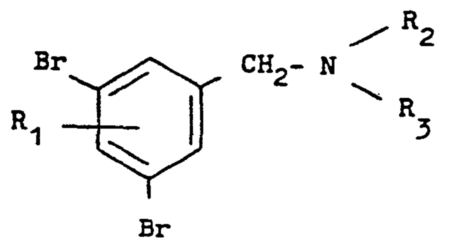

,(I)

in der

$R_1$ eine Hydroxygruppe in 2- oder 4-Stellung,

$R_2$ ein Wasserstoffatom oder eine Alkylgruppe mit 1 bis 3 Kohlenstoffatomen und

$R_3$ eine gegebenenfalls durch eine Hydroxygruppe substituierte Cyclohexylgruppe darstellen,

oder dessen physiologisch verträgliche Säureadditionssalze mit anorganischen oder organischen Säuren

und ein Antibioticum der Tetracyclingruppe, ein schwerer lösliches Penicillin, Erythromycin und seine Derivate, Spiramycin, Spiramycin-adipat, Tylosin, Tylosin-tartrat, Oleandomcycin, Chloramphenicol, Chloramphenicol-succinat, Thiamphenicol oder Thiamphenicol-glycinat,

ein Sulfonamid oder dessen Natriumsalz, auch als Kombination mit einem Agonisten sowie deren entsprechende Depotformen.

5. Kombination zur parenteralen Verabreichung ins Gewebe, enthaltend N-(2-Amino-3,5-dibrom-benzyl)-N-methyl-cyclohexylamin oder ein physiologisch verträgliches Säureadditionssalz dieser Verbindung und ein Oxytetracyclin-Langzeitpräparat, Oxytetracyclin-hydrochlorid-Langzeitpräparat, Rolitetracyclin, Doxycyclin,

ein schwerer lösliches Penicillin, z.b. Procain-Penicillin, Benethamin-Penicillin, Benzathin-Penicillin, ein Benzathin-Salz von Oxacillin, Cloxacillin oder Ampicillin,

Erythromycin oder eines seiner Derivat wie beispielsweise 9-Deoxy-11-deoxy-9,11-[imino[2-(2-methoxyäthoxy)-äthyliden]oxy]-(9F)-erythromycin, Erythromycin-lactobionat, Erythromycin-äthylsuccinat oder Erythromycin-glucoheptonat, Spiramycin, Spiramycin-adipat, Tylosin, Tylosin-tartrat, Oleandomycin, Thiamphenicol, Thiamphenicol-glycinat oder

ein Sulfonamid oder dessen Natriumsalz wie Sulfadiazin, Sulfadoxin, Sulfamethoxazol, Sulfadimethoxin, Sulfadimidin oder Sulfathiazol oder eine Kombination solcher mit einem Agonisten wie Trimethoprim, z.B. die Sulfadimidin-Sulfathiazol-Trimethoprim-Kombination.

6. Kombination zur parenteralen Verabreichung ins Gewebe, enthaltend N-(2-Amino-3,5-dibrom-benzyl)-trans-4-hydroxycyclohexylamin oder ein physiologisch verträgliches Säureadditionssalze dieser Verbindung und

ein Antibioticum der Tetracyclingruppe, z.B. Oxytetracyclin, Oxytetracyclin-hydrochlorid, Rolitetracyclin oder Doxycyclin,

ein schwerer lösliches Penicillin, z.B. Procain-Penicillin, Benethamin-Penicillin, Benzathin-Penicillin, einem Benzathin-Salz von Oxacillin, Cloxacillin oder Ampicillin,

Erythromycin oder eines seiner Derivate wie beispielsweise 9-Deoxy-11-deoxy-9,11-[imino[2-(2-methoxyäthoxy)-äthyliden]oxy]-(9F)-erythromycin, Erythromycin-lactobionat, Erythromycin-äthylsuccinat oder Erythromycin-glucoheptonat, Spiramycin, Spiramycin-adipat, Tylosin, Tylosin-tartrat, Oleandomycin, Chloramphenicol, Chloramphenicol-succinat, Thiamphenicol, Thiamphenicol-glycinat oder

ein Sulfonamid oder dessen Natriumsalz, z.B. Sulfadiazin, Sulfadoxin, Sulfamethoxazol, Sulfadimethoxin, Sulfadimidin oder Sulfathiazol, oder eine Kombination solcher mit einem Agonisten wie Trimethoprim, z.B. die Sulfadimidin-Sulfathiazol-Trimethoprim-Kombination,

sowie gegebenenfalls deren entsprechende Depotformen.

7. Kombination zur parenteralen Verabreichung ins Gewebe gemäß Anspruch 4 enthaltend N-(3,5-Dibrom-2-hydroxy-benzyl)-trans-4-hydroxy-cyclohexylamin oder dessen physiologisch verträgliche Säureadditionssalze mit anorganischen oder organischen Säuren.

8. Kombination gemäß den Ansprüchen 4 bis 7, dadurch gekennzeichnet, daß sie als antibakteriell wirksame Substanz

5—30 mg/kg Oxytetracyclin,
2—25 mg/kg Oxytetracyclin-hydrochlorid,
10—30 mg/kg Rolitetracyclin,
2—5 mg/kg Doxycyclin,
5—20 mg/kg Erythromycin und seine Derivate wie beispielsweise
5—20 mg/kg 9-Deoxy-11-deoxy-9,11-[imino[2-(2-methoxyäthoxy)-äthyliden]oxy]-(9F)-erythromycin,
10—50 mg/kg Spiramycin,
5—20 mg/kg Tylosin,
10—50 mg/kg Chloraphenicol,
10—50 mg/kg Thiamphenicol,
15—50 mg/kg Sulfadiazin,
15—30 mg/kg Sulfadiazin/Sulfathiazol-Trimethoprim,
15—30 mg/kg Sulfadoxin-trimethoprim,
2 000—20 000 I.E./kg Procain-Penicillin,
6 000—25 000 I.E./kg Benzathin-Penicillin,
2—15 mg/kg Ampicillin,
5—15 mg/kg Oxacillin oder Cloxacillin

enthält.

9. Kombination zur parenteralen Verabreichung ins Gewebe gemäß den Ansprüchen 4 und 7, enthaltend 0,2—1,2 mg/kg N-(3,5-Dibrom-2-hydroxy-benzyl)-trans-4-hydroxy-cyclohexylamin oder ein physiologisch verträgliches Säureadditionssalz dieser Verbindung mit einer anorganischen oder organischen Säure und 5 bis 20 mg/kg Tylosin.

10. Kombination zur parenteralen Verabreichung ins Gewebe gemäß den Ansprüchen 4 und 7, enthaltend 0,2—1,2 mg/kg N-(3,5-Dibrom-2-hydroxy-benzyl)-trans-4-hydroxy-cyclohexylamin oder ein physiologisch verträgliches Säureadditionssalz dieser Verbindung mit einer anorganischen oder organischen Säure und 5 bis 30 mg/kg Depot-Oxytetracyclin.

11. Kombination zur parenteralen Verabreichung ins Gewebe gemäß den Ansprüchen 4 und 7, enthaltend 0,2—1,2 mg/kg N-(3,5-Dibrom-2-hydroxy-benzyl)-trans-4-hydroxy-cyclohexylamin oder ein physiologisch verträgliches Säureadditionssalz dieser Verbindung mit einer anorganischen oder organischen Säure und 5 bis 20 mg/kg Erythromycin.

12. Kombination zur parenteralen Verabreichung ins Gewebe gemäß den Ansprüchen 4 und 7, enthaltend 0,2—1,2 mg/kg N-(3,5-Dibrom-2-hydroxy-benzyl)-trans-4-hydroxy-cyclohexylamin oder ein physiologisch verträgliches Säureadditionssalz dieser Verbindung mit einer anorganischen oder organischen Säure und 5 bis 50 mg/kg Sulfadiazin.

13. Kombination zur parenteralen Verabreichung ins Gewebe gemäß den Ansprüchen 4 und 7, enthaltend 0,2—1,2 mg/kg N-(3,5-Dibrom-2-hydroxy-benzyl)-trans-4-hydroxy-cyclohexylamin oder ein physiologisch verträgliches Säureadditionssalz dieser Verbindung mit einer anorganischen oder organischen Säure und 5 bis 20 mg/kg 9-Deoxy-11-deoxy-9,11-[imino[2-(2-methoxyäthoxy)äthyliden]-oxy]-(9F)-erythromycin.

14. Kombination zur parenteralen Verabreichung ins Gewebe gemäß den Ansprüchen 4 und 7, enthaltend 0,2—1,2 mg/kg N-(3,5-Dibrom-2-hydroxy-benzyl)-trans-4-hydroxy-cyclohexylamin oder ein physiologisch verträgliches Säureadditionssalz dieser Verbindung mit einer anorganischen oder organischen Säure und eine Sulfonamid-Agonistenkombination.

15. Kombination zur parenteralen Verabreichung ins Gewebe gemäß den Ansprüchen 4 und 7, enthaltend 0,2—1,2 mg/kg N-(3,5-Dibrom-2-hydroxy-benzyl)-trans-4-hydroxy-cyclohexylamin oder ein physiologisch verträgliches Säureadditionssalz dieser Verbindung mit einer anorganischen oder organischen Säure und 15 bis 30 mg/kg Sulfadiazin/Sulfathiazol/Trimethoprin.

16. Kombination zur parenteralen Verabreichung ins Gewebe gemäß den Ansprüchen 4 und 7, enthaltend 0,2—1,2 mg/kg N-(3,5-Dibrom-2-hydroxy-benzyl)-trans-4-hydroxy-cyclohexylamin oder ein physiologisch verträgliches Säureadditionssalz dieser Verbindung mit einer anorganischen oder organischen Säure und und 2 bis 15 mg/kg Ampicillin.

17. Kombination zur parenteralen Verabreichung ins Gewebe gemäß den Ansprüchen 4 und 7, enthaltend 0,2—1,2 mg/kg N-(3,5-Dibrom-2-hydroxy-benzyl)-trans-4-hydroxy-cyclohexylamin oder ein physiologisch verträgliches Säureadditionssalz dieser Verbindung mit einer anorganischen oder organischen Säure und 10 bis 50 mg/klg Chloramphenicol.

**Revendications**

1. Utilisation d'un dérivé de la benzylamine de formule générale

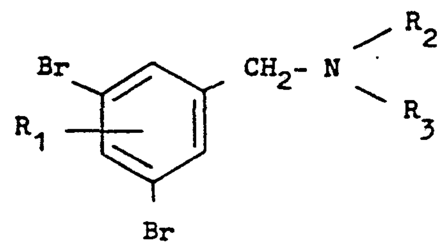

,(I)

dans laquelle

$R_1$ représente un groupe hydroxy en position 2 ou 4 ou un groupe amino en position 2,

$R_2$ représente un atome d'hydrogène ou un groupe alcoyle avec 1 à 3 atomes de carbone, et

$R_3$ représente un groupe cyclohexyle éventuellement substitué par un groupe hydroxy, ou d'un sel d'addition d'acide de celui-ci compatible physiologiquement avec un acide minéral ou organique pour la fabrication d'une préparation antibactérienne avec une résorption améliorée à administrer par voie parentérale dans le tissu, comprenant une substance ou combinaison à activité antibactérienne non résorbable de façon optimum.

2. Utilisation d'un dérivé de la benzylamine de formule générale I selon la revendication 1, caractérisée en ce que $R_1$ est tel que défini dans la revendication 1,

$R_2$ et $R_3$ représentent conjointement avec l'atome d'azote intermédiaire le groupe de N-méthyl-cyclohexylamino, N-éthyl-cyclohèxylamino, trans-4-hydroxy-cyclohexylamino ou cis-3-hydroxy-cyclohexylamino.

3. Utilisation d'un dérivé de la benzylamine de formule générale I selon la revendication 1, caractérisée en ce que

$R_1$ en position 2 représente le groupe hydroxy,

$R_2$ et $R_3$ conjointement avec l'atome d'azote intermédiaire représéntent le groupe trans-4-hydroxycyclohexylamino.

4. Combinaison pour l'administration parentérale dans le tissu, contenant un dérivé de la benzylamine de formule générale

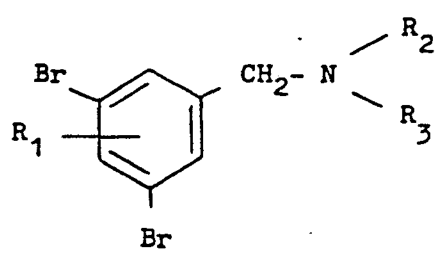

,(I)

dans laquelle

$R_1$ représente un groupe hydroxy en position 2 ou 4,

$R_2$ représente un atome d'hydrogène ou un groupe alcoyle avec 1 à 3 atomes de carbone et

$R_3$ représente un groupe cyclohexyle éventuellement substitué par un groupe hydroxy,

ou ses sels d'addition d'acide physiologiquement compatibles avec des acides minéraux ou organiques

et un antibiotique du groupe de la tétracycline, une pénicilline difficilement soluble, de l'érythromycine et ses dérivés, de la spiramycine, de l'adipate de spiramycine, de la tylosine, du tartrate de tylosine, de l'oléandomycine, du chloramphénicol, du succinate de chloramphénicol, du thiamphénicol ou du glycinate de thiamphénicol,

un sulfamide ou son sel de sodium, également en tant que combinaison avec un agoniste de même que ses formes retard correspondantes.

5. Combinaison pour l'administration parentérale dans le tissu contenant de la N-(2-amino-3,5-dibromobenzyl)-N-méthyl-cyclohexylamine ou un sel d'addition d'acide physiologiquement compatible de ce composé et une préparation longue durée d'oxytétracycline, une préparation longue durée de chlorhydrate d'oxytétracycline, de la rolitétracycline, de la doxycycline, une pénicilline plus difficilement soluble, par exemple de la procaïne-pénicilline, de la bénéthamine-pénicilline, de la benzathine-pénicilline, un sel de benzathine de l'oxacilline, de la cloxacilline ou de l'ampicilline, de l'érythromycine ou un de ses dérivés tels que par exemple la 9-déoxy-11-déoxy-9,11-[imino[2-(2-méthoxyéthoxy)-éthylidène]-oxy]-(9F)-érythromycine, le lactobionate d'érythromycine, léthylsuccinate d'érythromycine ou le glucoheptonate d'érhythromycine, la spiramycine, l'adipate de spiramycine, la tylosine, le tartrate de tylosine, l'oléandomycine, le thiamphénicol, le glycinate de thiamphénicol ou

un sulfamide ou son sel de sodium tel que la sulfadiazine, la sulfadoxine, le sulfaméthoxazol, la sulfadiméthoxine, la sulfadimidine ou le sulfathiazol ou une combinaison de ceux-ci avec un agoniste comme le triméthoprime, par exemple la combinaison sulfadimidine-sulfathiazol-triméthoprime.

6. Combinaison pour l'administration parentérale dans le tissus, comprenant de la N-(2-amino-3,5-dibromobenzyl)-trans-4-hydroxy-cyclohexylamine ou un sel d'addition d'acide physiologiquement compatible de ce composé et

un antibiotique du groupe de la tétracycline, par exemplè l'oxytétracycline, le chlorhydrate d'oxytétracycline, la rolitétracycline ou la doxycycline, une pénicilline difficilement soluble, par exemple la procaïne-pénicilline, la bénéthamine-pénicilline, la benzathine-pénicilline, un sel de benzathine de l'oxacilline, de la cloxacilline ou de l'ampicilline,

de l'érythromycine ou l'une de ses dérivés comme par exemple la 9-déoxy-11-déoxy-9,11-[imino[2-(2-méthoxyéthoxy)-éthylidène]-oxy]-(9F)-érythromycine, le lactobionate d'érythromycine, l'éthylsuccinate d'érythromycine ou le glucoheptonate d'érythromycine, la spiramycine, l'adipate de spiramycine, la tylosine, le tartrate de tylosine, l'oléandomycine, le chloramphénicol, le succinate de chloramphénicol, le thiamphénicol, le glycinate de thiamphénicol ou

un sulfamide ou son sel de sodium, par exemple la sulfadiazine, la sulfadoxine, le sulfaméthoxazol, la sulfadiméthoxine, la sulfadimidine ou le sulfathiazol, ou une combinaison de ceux-ci avec un agoniste comme le triméthoprime par exemple la combinaison sulfadimidine-sulfathiazol-triméthoprime,

de même qu'éventuellement leurs formes retard correspondantes.

7. Combinaison pour l'administration parentérale dans le tissu selon la revendication 4 contenant de la N-(3,5-dibromo-2-hydroxy-benzyl)-trans-4-hydroxy-cyclohexylamine ou ses sels d'addition d'acides compatibles physiologiquement avec des acides minéraux ou organiques.

8. Combinaison selon les revendications 4 à 7, caractérisée en ce qu'elle contient en tant que substance à activité antibactérienne:

5—30 mg/kg d'oxytétracycline,
2—25 mg/kg d'oxytétracycline,
2—25 mg/kg de chlorhydrate d'oxytétracycline,
10—30 mg/kg de rolitétracycline,
2—5 mg/kg de doxycycline,
5—20 mg/kg d'erythromycine et ses dérivés comme par exemple,
5—20 mg/kg de 9-déoxo-11-déoxy-9,11-[imino][2-(2-méthoxyéthoxy)-éthylidène]oxy]-(9F)-érythromycine,
10—50 mg/kg de spiramycine,
5—20 de tylosine,
10—50 mg/kg de chloramphénicol,
10—50 mg/kg de thiamphénicol,
15—50 mg/kg de sulfadiazine,
15—30 mg/kg de sulfadiazine/sulfathiazol-triméthoprime,
15—30 mg/kg de sulfadoxine-triméthoprime,
2 000—20 000 U.I./kg procaïne-pénicilline,
6 000—25 000 U.I./kg benzathine-pénicilline,
2—15 mg/kg d'ampicilline,
5—15 mg/kg d'oxacilline ou de cloxacilline.

9. Combinaison pour l'administration parentérale dans le tissu selon les revendications 4 et 7, contenant 0,2—1,2 mg/kg de N-(3,5-dibromo-2-hydroxy-benzyl)-trans-4-hydroxy-cyclohexylamine ou un sel d'addition d'acide de ce composé compatible physiologiquement, avec un acide minéral ou organique et 5 à 20 mg/kg de tylosine.

10. Combinaison pour l'administration parentérale dans le tissu selon les revendications 4 et 7, contenant 0,2—1,2 mg/kg de N-(3,5-dibromo-2-hydroxy-benzyl)-trans-4-hydroxy-cyclohexylamine ou un sel d'addition d'acide de ce composé compatible physiologiquement, avec un acide minéral ou organique et 5 à 30 mg/kg d'oxytétracycline retard.

11. Combinaison pour l'administration parentérale dans le tissu selon les revendications 4 et 7, contenant 0,2—1,2 mg/kg de N-(3,5-dibromo-2-hydroxy-benzyl)-trans-4-hydroxy-cyclohexylamine ou un sel d'addition d'acide de ce composé compatible physiologiquement, avec un acide minéral ou organique et 5 à 20 mg/kg d'érythromycine.

12. Combinaison pour l'administration parentérale dans le tissu selon les revendications 4 et 7, contenant 0,2—1,2 mg/kg de N-(3,5-dibromo-2-hydroxy-benzyl)-trans-4-hydroxy-cyclohexylamine ou un sel d'addition d'acide de ce composé compatible physiologiquement, avec un acide minéral ou organique et 15 à 50 mg/kg de sulfadiazine.

13. Combinaison pour l'administration parentérale dans le tissu selon les revendications 4 et 7, contenant 0,2—1,2 mg/kg de N-(3,5-dibromo-2-hydroxy-benzyl)-trans-4-hydroxy-cyclohexylamine ou un sel d'addition d'acide de ce composé compatible physiologiquement, avec un acide minéral ou organique et 5 à 20 mg/kg 9-déoxo-11-déoxy-9,11-[imino[2-(2-méthoxyéthoxy)-éthylidène)oxy]-(9F)-érythromycine.

14. Combinaison pour l'administration parentérale dans le tissu selon les revendications 4 et 7, contenant 0,2—1,2 mg/kg de N-(3,5-dibromo-2-hydroxy-benzyl)-trans-4-hydroxy-cyclohexylamine ou un

sel d'addition d'acide de ce composé compatible physiologiquement, avec un acide minéral ou organique et une combinaison sulfamide-agoniste.

15. Combinaison pour l'administration parentérale dans le tissu selon les revendications 4 et 7, contenant 0,2—1,2 mg/kg de N-(3,5-dibromo-2-hydroxy-benzyl)-trans-4-hydroxy-cyclohexylamine ou un sel d'addition d'acide de ce composé compatible physiologiquement, avec un acide minéral ou organique et 15 à 30 mg/kg de sulfadiazine/sulfathiazol/triméthoprime.

16. Combinaison pour l'administration parentérale dans le tissu selon les revendications 4 et 7, contenant 0,2—1,2 mg/kg de N-(3,5-dibromo-2-hydroxy-benzyl)-trans-4-hydroxy-cyclohexylamine ou un sel d'addition d'acide de ce composé compatible physiologiquement, avec un acide minéral ou organique et 2 à 15 mg/kg d'ampicilline.

17. Combinaison pour l'administration parentérale dans le tissu selon les revendications 4 et 7, contenant 0,2—1,2 mg/kg de N-(3,5-dibromo-2-hydroxy-benzyl)-trans-4-hydroxy-cyclohexylamine ou un sel d'addition d'acide de ce composé compatible physiologiquement, avec un acide minéral ou organique et 10 à 50 mg/kg de chloramphénicol.

**Claims**

1. Use of a benzylamine derivative of general formula

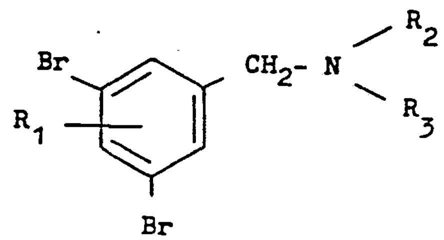

,(I)

wherein

$R_1$ represents a hydroxy group in the 2- or 4-position or an amino group in the 2-position,

$R_2$ represents a hydrogen atom or an alkyl group with 1 to 3 carbon atoms and

$R_3$ represents a cyclohexyl group optionally substituted by a hydroxy group, or a physiologically acceptable acid addition salt thereof with an inorganic or organic acid for preparing an antibacterial preparation with improved resorption, intended for parenteral administration into the tissue, this antibacterial preparation containing an antibacterially active substance or combination which does not have optimum resorbability.

2. Use of a benzylamine derivative of general formula I as claimed in claim 1, characterised in that $R_1$ is defined as in claim 1, $R_2$ and $R_3$ together with the nitrogen atom between them represent an N-methyl-cyclohexylamino, N-ethylcyclohexylamino, trans-4-hydroxy-cyclohexylamino or cis-3-hydroxy-cyclohexylamino group.

3. Use of a benzylamine derivative of general formula I as claimed in claim 1, characterised in that

$R_1$ in the 2 position represents a hydrogen group,

$R_2$ and $R_3$ together with the nitrogen atom between them represent a trans-4-hydroxy-cyclohexylamino group.

4. Combination for parenteral administration into the tissue, containing

a benzylamine derivative of general formula

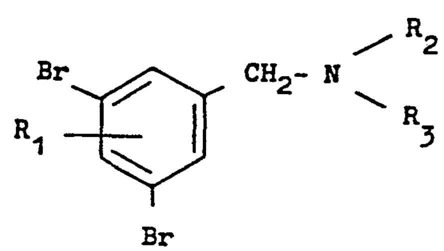

,(I)

wherein

$R_1$ represents a hydroxy group in the 2 or 4 position,

$R_2$ represents a hydrogen atom or an alkyl group with 1 to 3 carbon atoms and

$R_3$ represents a cyclohexyl group optionally substituted by a hydroxy group,

or the physiologically acceptable acid addition salts thereof with inorganic or organic acids and an antibiotic of the tetracycline group, a difficultly soluble penicillin, erythromycin and the derivatives thereof, spiramycin, spiramycin adipate, tylosine, tylosine-tartrate, oleandomycin, chloramphenicol, chloramphenicol-succinate, thiamphenicol or thiamphenicol-glycinate,

a sulphonamide or the sodium salt thereof, also as a combination with an agonist and the corresponding delayed-release forms.

5. Combination for parenteral administration into the tissue, containing N-(2-amino-3,5-dibromo-benzyl)-N-methyl-cyclohexylamine or a physiologically acceptable acid addition salt thereof and a long-term oxytetracycline preparation, long-term oxytetracycline hydrochloride preparation, rolitetracycline, doxycycline,

a difficultly soluble penicillin, e.g. procaine penicillin, benethamine penicillin, benzathine penicillin, a benzathine salt of oxacillin, cloxacillin or ampicillin,

erythromycin or one of the derivatives thereof, such as 9-deoxy-11-deoxy-9,11-[imino[2-(2-methoxyethoxy)ethylidene]-oxy]-(9F)-erythromycin, erythromycin lactobionate, erythromycin ethylsuccinate or erythromycin glucoheptonate, spiramycin, spiramycin adipate, tylosine, tylosine tartrate, oleandomycin, thiamphenicol, thiamphenicol glycinate or

a sulphonamide or the sodium salt thereof such as sulphadiazine, sulphadoxine, sulphamethoxazole, sulphadimethoxine, sulphadimidine or sulphathiazole or a combination thereof with an agonist such as trimethoprim, e.g. a combination of sulphadimidine-sulphathiazole-trimethoprim.

6. Combination for parenteral administration into the tissue, containing N-(2-amino-3,5-dibromobenzyl)-trans-4-hydroxycyclohexylamine or a physiologically acceptable acid addition salt thereof and

an antibiotic of the tetracycline group, e.g. oxytetracycline, oxytetracycline hydrochloride, rolitetracycline or doxycycline, a difficultly soluble penicillin, e.g. procaine penicillin, benethamine penicillin, benzathine penicillin, a benzathine salt of oxacillin, cloxacillin or ampicillin,

erythromycin or one of the derivatives thereof such as 9-deoxy-11-deoxy-9,11-[imino[2-(2-methoxyethoxy)ethylidene]-oxy]-(9F)-erythromycin, erythromycin lactobionate, erythromycin ethylsuccinate or erythromycin glucoheptonate, spiramycin, spiramycin adipate, tylosin, tylosin tartrate, oleandomycin, chloramphenicol, chloramphenicol succinate, thiamphenicol, thiamphenicol glycinate or

a sulphonamide or the sodium salt thereof, e.g. sulphadiazine, sulphadoxine, sulphamethoxazole, sulphadimethoxine, sulphadimidine or sulphathiazole, or a combination thereof with an agonist such as trimethoprim, e.g. a combination of sulphadimidine-sulphathiazole-trimethoprim,

and optionally the corresponding delayed-release forms.

7. Combination for parenteral administration into the tissue as claimed in claim 4, containing N-(3,5-dibromo-2-hydroxy-benzyl)-trans-4-hydroxycyclohexylamine or the physiologically acceptable acid addition salts thereof with inorganic or organic acids.

8. Combination as claimed in claims 4 to 7, characterised in that it contains as bacterially active substance

5—30 mg/kg of oxytetracycline,
2—25 mg/kg of oxytetracycline hydrochloride,
10—30 mg/kg of rolitetracycline,
2—5 mg/kg of doxycycline,
5—20 mg/kg of erythromycin and the derivatives thereof such as
5—20 mg/kg of 9-deoxy-11-deoxy-9,11-[imino[2-(2-methoxyethoxy)-ethylidene]oxy]-(9F)-erythromycin,
10—50 mg/kg of spiramycin,
5—20 mg/kg of tylosin,
10—50 mg/kg of chloramphenicol,
10—50 mg/kg of thiamphenicol,
15—50 mg/kg of sulphadiazine,
15—30 mg/kg of sulphadiazine/sulphathiazole/trimethoprim,
15—30 mg/kg of sulphadoxin/trimethoprim,
2,000—20,000 I.U./kg of procaine penicillin,
6,000—25,000 I.U./kg of benzathine penicillin,
2—15 mg/kg of ampicillin, or
5—15 mg/kg of oxacillin or cloxacillin.

9. Combination for parenteral administration into the tissue as claimed in claims 4 and 7, containing 0.2 to 1.2 mg/kg of N-(3,5-dibromo-2-hydroxy-benzyl)-trans-4-hydroxy-cyclohexylamine or a physiologically acceptable acid addition salt of this compound with an inorganic acid and 5 to 20 mg/kg of tylosin.

10. Combination for parenteral administration into the tissue as claimed in claims 4 and 7, containing 0.2 to 1.2 mg/kg of N-(3,5-dibromo-2-hydroxy-benzyl)-trans-4-hydroxy-cyclohexylamine or a physiologically acceptable acid addition salt of this compound with an inorganic or organic acid and 5 to 30 mg/kg of delayed-release oxytetracycline.

11. Combination for parenteral administration into the tissue as claimed in claims 4 and 7, containing 0.2 to 1.2 mg/kg of N-(3,5-dibromo-2-hydroxy-benzyl)-trans-4-hydroxy-cyclohexylamine or a physiologically acceptable acid addition salt of this compound with an inorganic or organic acid and 5 to 20 mg/kg of erythromycin.

12. Combination for parenteral administration into the tissue as claimed in claims 4 and 7, containing 0.2 to 1.2 mg/kg of N-(3,5-dibromo-2-hydroxy-benzyl)-trans-4-hydroxy-cyclohexylamine or a physiologically acceptable acid addition salt of this compound with an inorganic or organic acid and 15 to 50 mg/kg of sulphadiazine.

13. Combination for parenteral administration into the tissue as claimed in claims 4 and 7, containing 0.2 to 1.2 mg/kg of N-(3,5-dibromo-2-hydroxy-benzyl)-trans-4-hydroxy-cyclohexylamine or a physiologically acceptable acid addition salt of this compound with an inorganic or organic acid and 5 to 20 mg/kg of 9-deoxo-11-deoxy-9,11-[imino[2-(2-methoxyethoxy)ethylidene]-oxy]-(9f)-erythromycin.

14. Combination for parenteral administration into the tissue as claimed in claims 4 and 7, containing 0.2 to 1.2 mg/kg of N-(3,5-dibromo-2-hydroxy-benzyl)-trans-4-hydroxy-cyclohexylamine or a physiologically acceptable acid addition salt of this compound with an inorganic or organic acid and a sulphonamide-agonist combination.

15. Combination for parenteral administration into the tissue as claimed in claims 4 and 7, containing 0.2 to 1.2 mg/kg of N-(3,5-dibromo-2-hydroxy-benzyl)-trans-4-hydroxy-cyclohexylamine or a physiologically acceptable acid addition salt of this compound with an inorganic or organic acid and 15 to 30 mg/kg of sulphadiazine/sulphathiazole/trimethoprim.

16. Combination for parenteral administration into the tissue as claimed in claims 4 and 7, containing 0.2 to 1.2 mg/kg of N-(3,5-dibromo-2-hydroxy-benzyl)-trans-4-hydroxy-cyclohexylamine or a physiologically acceptable acid addition salt of this compound with an inorganic or organic acid and 2 to 15 mg/kg of ampicillin.

17. Combination for parenteral administration into the tissue as claimed in claims 4 and 7, containing 0.2 to 1.2 mg/kg of N-(3,5-dibromo-2-hydroxy-benzyl)-trans-4-hydroxy-cyclohexylamine or a physiologically acceptable acid addition salt of this compound with an inorganic or organic acid and 10 to 50 mg/kg of chloramphenicol.